# DEMANDE DE BREVET EUROPEEN

(11) **EP 0 636 387 A1**
(43) Date de publication de la demande: **01.02.1995**
(21) Numéro de dépôt: 94440049.8
(22) Date de dépôt: 28.07.1994
(51) Int. Cl.: A61N 5/00, A61N 5/02

(54) **Procédé de calibration et d'étalonnage d'un radiomètre pour la mesure de températures**

(30) Priorité: 29.07.1993 FR 9309525
(71) Demandeur: SADIS BRUKER SPECTROSPIN, SOCIETE ANONYME DE DIFFUSION DE L'INSTRUMENTATION SCIENTIFIQUE BRUKER SPECTROSPIN (S.A. à Direct.), F-67160 Wissembourg (FR)
(72) Inventeur: Mabire, Jean-Pierre, F-67500 Haguenau (FR); Ringeisen, Victor, F-67160 Riedseltz (FR)
(74) Mandataire: Nuss, Pierre

(57) **Abrégé**

La présente invention a pour objet un procédé de calibration et d'étalonnage d'un radiomètre pour la mesure de températures.

Procédé caractérisé en ce qu'il consiste à mettre en contact l'antenne radiométrique, ensemble avec la portion d'un dispositif de thermostatisation entourant ladite antenne, avec un milieu, notamment liquide, à température connue, puis à prendre en compte la valeur de température mesurée par ledit radiomètre et, enfin, à répéter au moins une fois les deux opérations précédentes en mettant en contact ladite antenne radiométrique et ladite portion du dispositif de thermostatisation avec un milieu à une température connue différente.

## Description

La présente invention concerne le réglage d'instruments de mesure, plus particulièrement d'instruments de mesure de la température dans le cadre de la thermothérapie micro-onde de la prostate, et a pour objet un procédé de calibration et d'étalonnage d'un radiomètre pour la mesure de températures.

Un radiomètre constitue un dispositif de mesure de température non invasif, prenant en compte, au moyen d'une antenne, le bruit thermique du milieu dont on veut déterminer la température, ce dispositif réalisant une mesure relative et devant donc être étalonné et calibré avant toute mise en oeuvre.

Actuellement, les appareils de traitement par thermothérapie micro-onde de la prostate, dont notamment l'appareil de la demanderesse connue sous la désignation "PROSTCARE", comporte une antenne radiométrique confondue avec l'antenne d'émission de micro-ondes et thermostatée en permanence par de l'eau circulant autour de ladite antenne et maintenue à une température constante.

Par conséquent, le radiomètre est sensible, lors de sa mise en oeuvre, à deux facteurs variables, à savoir, d'une part, la température de l'eau de thermostatisation circulant autour de l'antenne radiométrique et, d'autre part, la température du milieu à mesurer.

La valeur de la température de l'eau circulant de thermostatisation est fixée et connue, et a une grande influence sur la valeur de la température mesurée par le radiomètre, du fait qu'elle se trouve en contact direct avec l'antenne radiométrique autour de laquelle elle circule.

Or, l'étalonnage et la calibration du radiomètre s'effectuent actuellement en plongeant l'antenne radiométrique seule dans au moins deux bains à températures connues différentes, entraînant une calibration absolue dudit radiomètre.

Toutefois, il y a alors lieu, pour chaque mesure de température pratique de corriger la valeur mesurée en intégrant l'influence de la présence de l'eau circulante de thermostatisation, l'antenne radiométrique étant mise en contact avec le milieu à mesurer ensemble avec l'eau thermostatée circulant autour de cette dernière.

La présente invention a notamment pour but de pallier cet inconvénient.

A cet effet, elle a pour objet un procédé de calibration et d'étalonnage d'un radiomètre pour la mesure de températures, notamment d'un système de traitement par thermothérapie micro-onde, caractérisé en ce qu'il consiste à mettre en contact l'antenne radiométrique, ensemble avec la portion d'un dispositif de thermostatisation entourant ladite antenne, avec un milieu, notamment liquide, à température connue, puis à prendre en compte la valeur de température mesurée par ledit radiomètre et, enfin, à répéter au moins une fois les deux opérations précédentes en mettant en contact ladite antenne radiométrique et ladite portion du dispositif de thermostatisation avec un milieu à une température connue différente.

Ainsi, l'influence de l'eau circulante de thermostatisation est prise en compte automatiquement, par intégration physique, lors de l'étalonnage et de la calibration dudit radiomètre au moyen d'au moins deux points de température connus et l'utilisateur pourra utiliser, lors de mesures pratiques après étalonnage, directement la valeur de la température relevée et affichée par ledit radiomètre, ce sans aucune correction.

De manière avantageuse, l'antenne radiométrique et la portion du dispositif de thermostatisation entourant cette dernière sont mises en contact avec les milieux à températures connues dans des conditions identiques à celles existant lors de la mise en contact dudit ensemble antenne radiométrique/dispositif de thermostatisation avec le milieu dont on veut mesurer la température.

Il conviendra ainsi de veiller notamment à conserver une surface d'interface identique, entre le milieu environnant et l'ensemble antenne radiométrique/portion du dispositif de thermostatisation, lors de l'étalonnage et de la calibration et lors des mesures pratiques de température.

Dans le cas d'appareils de thermothérapie micro-onde de la prostate, notamment celui de la demanderesse, le dispositif de thermostatisation consiste avantageusement en une gaine de fluide circulant autour de ladite antenne radiométrique et maintenue à une température constante.

Le fluide pourra notamment être de l'eau, maintenue, par exemple, à une température de 20°C.

Selon un mode de réalisation préféré du procédé d'étalonnage et de calibration conforme à l'invention, l'antenne radiométrique et la portion du dispositif de thermostatisation entourant cette dernière sont immergées, sur une longueur prédéterminée, dans des milieux à températures connues consistant en des liquides.

Bien entendu, l'invention n'est pas limitée au mode de réalisation décrit ci-dessus. Des modifications restent possibles, notamment du point de vue de la constitution des divers éléments, ou par substitution d'équivalents techniques, sans sortir pour autant du domaine de protection de l'invention.

## Revendications

1. Procédé de calibration et d'étalonnage d'un radiomètre pour la mesure de températures, notamment d'un système de traitement par thermothérapie micro-onde, caractérisé en ce qu'il consiste à mettre en contact l'antenne radiométrique, ensemble avec la portion d'un dispositif de thermostatisation entourant ladite antenne, avec un milieu, notamment liquide, à température connue, puis à prendre en compte la valeur de température mesurée par ledit radiomètre et, enfin, à répéter au moins une fois les deux opérations précédentes en mettant en contact ladite antenne radiométrique et ladite portion du dispositif de thermostatisation avec un milieu à une température connue différente.

2. Procédé selon la revendication 1, caractérisé en ce que l'antenne radiométrique et la portion du dispositif de thermostatisation entourant cette dernière sont mises en contact avec les milieux à températures connues dans des conditions identiques à celles existant lors de la mise en contact dudit ensemble antenne radiométrique/dispositif de thermostatisation avec le milieu dont on veut mesurer la température.

3. Procédé selon l'une quelconque des revendications 1 et 2, caractérisé en ce que le dispositif de thermostatisation consiste en une gaine de fluide circulant autour de ladite antenne radiométrique et maintenue à une température constante.

4. Procédé selon la revendication 3, caractérisé en ce que le fluide est de l'eau maintenue, par exemple, à une température de 20°C.

5. Procédé selon l'une quelconque des revendications 1 à 4, caractérisé en ce que l'antenne radiométrique et la portion du dispositif de thermostatisation entourant cette dernière sont immergées, sur une longueur prédéterminée. dans des milieux à températures connues consistant en des liquides.
